# EUROPÄISCHE PATENTSCHRIFT

(11) **EP 2 954 915 B1**
(45) Veröffentlichungstag und Bekanntmachung des Hinweises auf die Patenterteilung: **26.09.2018**
(21) Anmeldenummer: 15170959.9
(22) Anmeldetag: 08.06.2015
(51) Int. Cl.: A61M 5/31, A61J 1/00

(54) **BEHÄLTER BESTEHEND AUS MEHREREN KOMPONENTEN**
CONTAINER MADE OF SEVERAL COMPONENTS
RÉCIPIENT COMPOSÉ DE PLUSIEURS COMPOSANTS

(30) Priorität: 10.06.2014 DE 102014211018
(43) Veröffentlichungstag der Anmeldung: 16.12.2015
(73) Patentinhaber: SCHOTT Schweiz AG, 9001 St. Gallen (CH)
(72) Erfinder: Küçük, Mustafa, 9422 Staad (CH); Bamberg, Klaus, 9524 Zuzwil SG (CH); Hoppe, Bernd, 55218 Ingelheim (DE)
(74) Vertreter: Westphal, Mussgnug & Partner Patentanwälte mbB

(56) Entgegenhaltungen:
- JP-A- 2009 219 855
- US-A- 3 885 297
- US-A- 5 836 919
- US-B2- 7 727 203

## Beschreibung

Die vorliegende Erfindung betrifft einen Behälter zum Lagern und/oder Applizieren einer Substanz, umfassend einen Glaskörper, der einen Hohlraum umschließt und ein distales Ende mit einer ersten Öffnung aufweist, und einen oder mehrere am Glaskörper angebrachte Anschlusskörper, die aus einem Kunststoff bestehen oder diesen umfassen, wobei einer der Anschlusskörper am distalen Ende angeordnet ist. Als Beispiel für Substanzen, die im erfindungsgemäßen Behälter gelagert werden können, seien sowohl pastöse, flüssige als auch gasförmige Substanzen und Gemische sowie Dispersionen und Emulsionen genannt. Da Glas eine hohe Inertie gegenüber einem Großteil der gebräuchlichen Chemikalien und pharmazeutischen Substanzen und eine hohe Diffusionsdichtigkeit aufweist, eignet es sich besonders zum Lagern von pharmazeutischen Substanzen. Aufgrund der hohen Diffusionsdichtigkeit sind Permeationsverluste während der Lagerung gering, was insbesondere bei hochwertigen pharmazeutischen Substanzen ein wichtiger Aspekt ist.

Insbesondere bei modernen pharmazeutischen Wirkstoffen, die sehr teuer, hochwirksam und sehr empfindlich sind, geht man mehr und mehr dazu über, vorgefüllte Spritzen oder Karpulen einzusetzen, wobei sich aus den oben genannten Gründen Spritzen aus Glas anbieten. Mit vorgefüllten Spritzen ist es nicht mehr notwendig, den Wirkstoff von einem Behälter in einen anderen Behälter zu überführen. Vielmehr ist die vorgefüllte Spritze sofort nach dem Entpacken gebrauchsfertig. Neben der Zeitersparnis für den Arzt oder die Krankenschwester kommt der Vorteil hinzu, dass Verluste, die beim Überführen von einem in den anderen Behälter häufig auftreten, vermieden werden. Zudem besteht beim Überführen das Risiko einer Infektion oder einer Kontamination der Substanz und/oder der Spritze. Dieses Risiko wird mit vorgefüllten Spritzen deutlich verringert.

Spritzen weisen dort, wo die Kanüle angeschlossen wird, üblicherweise einen dünnen Kanal auf. Bei Glasspritzen wird dieser dünne Kanal unter Verwendung eines Wolframstifts gefertigt, der während des Umformprozesses als Formwerkzeug dient. Das erhitzte Glas wird im Bereich des Kanals auf die Mantelfläche des Wolframstifts gedrückt. Nach Abschluss des Umformprozesses wird der Wolframstift aus der Spritze entnommen und es bleibt der Kanal zurück.

Ohne Verwendung des Wolframstifts kann der dünne Kanal nicht in der gewünschten Genauigkeit gefertigt werden. Zudem besteht die Gefahr, dass der Kanal ohne die Verwendung des Wolframstifts beim Umformen verschlossen wird. Der Stift ist deshalb aus Wolfram, weil er die hohen Temperaturen, auf die das Glas beim Umformen gebracht werden muss, um die notwendige Viskosität zu erreichen, ohne wesentliche chemischen oder mechanischen Veränderungen übersteht. Nachteilig hieran ist aber, dass beim Entnehmen des Wolframstifts ein Abrieb entsteht, so dass Wolfram-Rückstände in der Spritze zurückbleiben, die in die gelagerte Substanz migrieren können. Dies ist insbesondere dann unerwünscht, wenn pharmazeutische Wirkstoffe in der Spritze gelagert werden.

Zudem weisen Spritzen relativ komplizierte Geometrien auf, um Kanülen oder Schläuche zum Applizieren der pharmazeutischen Substanzen anschließen zu können. Als Beispiel sei an dieser Stelle ein Luer-Lock-Anschluss erwähnt, der nur mit erheblichem Aufwand aus Glas zu fertigen ist und sich für die Massenproduktion unter Verwendung mit Glas nicht eignet. Aus diesen Gründen geht man dazu über, vorgefüllte Spritzen aus Kunststoff zu verwenden, wodurch es möglich ist, beispielsweise mittels eines Spritzgussverfahrens auch kompliziertere Geometrien mit relativ geringem Aufwand herzustellen. Allerdings sind die Formwerkzeuge zum Herstellen der Kunststoffspritzen relativ aufwendig, so dass sich die Herstellung von Kunststoffspritzen meistens nur bei hohen Stückzahlen rechnet. Zudem muss für jede Spritzengröße ein eigenes Formwerkzeug bereitgestellt werden. Allerdings weisen Kunststoffe bei weitem nicht dieselbe Inertie wie Glas auf, so dass nur eine begrenzte Anzahl von pharmazeutischen Substanzen in Kunststoffspritzen gelagert werden kann. Darüber hinaus ist die Lagerdauer der pharmazeutischen Substanzen in Kunststoffspritzen aufgrund der niedrigeren Diffusionsdichtigkeit geringer als bei Glasspritzen. Weiterhin kommt es bei der Lagerung zu nicht unerheblichen Permeationsverlusten. Diese Permeationsverluste können mit speziellen Beschichtungen verringert werden, die aber aufwendig herzustellen sind.

Die US 2010/0280414 A1 zeigt einerseits einen Behälter zum Sammeln eines biologischen Probe wie Blut, der mit einem stopfenförmigen Anschlusskörper verschlossen ist, der ausschließlich über die seitliche Innenfläche am Behälter befestigt wird. Es wird ein Reibschluss erzeugt. Andererseits zeigt die US 2010/0280414 A1 einen Behälter, bei dem einer Nadelhalter über eine form- oder reibschlüssige Verbindung mit der Außenfläche eines Zylinders in Wechselwirkung tritt und so am Behälter befestigt wird.

Die DE 101 02 054 zeigt eine Ampulle mit einer Sollbruchstelle, die mit einem Stopfen verschlossen werden kann, der aber auch nur über Innenfläche des Behälters an der Ampulle befestigt werden kann.

Beide Behälter eignen sich nicht zum Applizieren von zähflüssigen Substanzen, da die Verbindung zwischen dem Behälter und den betreffenden Anschlusskörpern die dabei auftretenden Kräfte nicht aufnehmen kann.

Die US 3 885 297 B1 und die JP 2009/219855 offenbaren eine Spritze mit einem hohlzylindrischen Glaskörper, an dessen erstem und zweitem Ende jeweils ein Anschlusskörper beispielsweise aus thermoplastischem Kunststoff mittels eines Formschlusses angeschlossen ist. Hierzu weist der hohlzylindrische Glaskörper and den Stirnflächen der ersten und zweiten Enden jeweils einen torusförmigen Wulst auf, über welchen der Formschluss mit den Anschlusskörpern ausgebildet wird.

Die US 5 836 919 B1 offenbart eine Spritze mit einem hohlzylindrischen Glaskörper, an dessen erstem und zweitem Ende jeweils ein Anschlusskörper aus thermoplastischem Kunststoff mittels eines Reibschlusses angeschlossen ist. Hierzu sind die Anschlusskörper elastisch ausgestaltet und weisen je nach Ausführungsbeispiel Rippen auf, die sich an die äußere Oberfläche des Glaskörpers anlegen, um den Reibschluss auszubilden. Auch die US 7 727 203 B2 zeigt eine Spritze mit einem hohlzylindrischen Glaskörper, an dessen erstem und zweitem Ende jeweils ein Anschlusskörper mittels eines Reibschlusses befestigt ist.

Bei der form- und/oder reibschlüssigen Verbindung der Anschlusskörper mit dem Glaskörper handelt es sich nicht um eine unlösbare Verbindung, da sich beispielsweise durch Relaxationsvorgänge im Kunststoff die Reibung zwischen den Anschlusskörpern und dem Glaskörper im Laufe der Zeit verringert. Hierdurch kann die reibschlüssige Verbindung versagen. Ähnliche Veränderungen des Kunststoffs können bei einem Formschluss zu einem Versagen der Verbindung führen.

Aufgabe der vorliegenden Erfindung ist es daher, den Behälter der eingangs genannten Art zum Lagern einer Substanz so weiterzuentwickeln, dass er einerseits eine hohe Diffusionsdichtigkeit und Inertie aufweist und andererseits auch dann günstig zu fertigen ist, wenn der Anschlusskörper eine komplexe Geometrie aufweist.

Gelöst wird die Aufgabe durch einen Behälter nach Anspruch 1. Vorteilhafte Weiterbildungen ergeben sich aus den Unteransprüchen.

Erfindungsgemäß ist der Kunststoff ein thermoplastischer oder duroplastischer Kunststoff und sind der Glaskörper und der Anschlusskörper unter Verwendung einer mittels eines Fügeverfahrens bereitgestellten Verbindung miteinander verbunden, wobei der am distalen Ende angeordnete Anschlusskörper einen mit der ersten Öffnung kommunizierenden Durchtrittskanal umfasst. Die Öffnungen werden von Stirnflächen und von unmittelbar angrenzenden Flächen des Glaskörpers umschlossen, wobei der oder die Anschlusskörper über die Stirnflächen und über die unmittelbar angrenzenden Flächen am Glaskörper befestigt sind. Die die Verbindung zwischen dem Glaskörper und dem oder den Anschlusskörpern ist unlösbar, wobei die unmittelbar angrenzenden Flächen der Teil der inneren Mantelfläche des Glaskörpers sind, der sich den Stirnflächen anschließt.

Unter einem thermoplastischen Kunststoff sind Kunststoffe zu verstehen, die sich in einem bestimmten Temperaturbereich verformen lassen. Zur Bereitstellung der Verbindung kann ein thermisches Fügeverfahren zum Einsatz kommen, bei dem der thermoplastische Kunststoff erwärmt und so umgeformt wird, dass eine Verbindung mit dem Glaskörper bereitgestellt wird. Das Glas selbst wird dabei nicht umgeformt.

Bei duroplastischen Kunststoffen kann der Anschlusskörper in einem Spritzgusswerkzeug geformt werden, wobei ein Zweikomponentensystem zum Einsatz kommen kann, welches die Aushärtung des duroplastischen Kunststoffs bewirkt. Beispielsweise kann ein Epoxidharz zum Einsatz kommen. Bei diesem Fügeverfahren wird die Verbindung mit dem Glaskörper beim Aushärten des duroplastischen Kunststoffs ausgebildet. Auch hier bleibt das Glas unverändert. Beiden Fügeverfahren ist gemein, dass sie nur auf den Anschlusskörper einwirken und die Verbindung durch Änderung der Viskosität des gesamten Anschlusskörpers oder Teile hiervon bereitgestellt wird. Zudem wird die Form des Anschlusskörpers beim Fügen zumindest minimal verändert.

Der erfindungsgemäße Behälter vereint die oben bereits ausgeführten Vorteile des Glases, insbesondere seine Inertie und Diffusionsdichtigkeit, mit den Vorteilen des Kunststoffs, nämlich insbesondere die vereinfachte Formgebung, so dass die Anschlusskörper ohne großen Mehraufwand auch mit komplexen Geometrien versehen werden können. Dabei weist der Glaskörper des erfindungsgemäßen Behälters eine einfach zu fertigende Geometrie auf und macht dabei den Großteil der Kontaktfläche mit der gelagerten Substanz aus. Im Vergleich zu einem Behälter, der komplett aus Glas gefertigt ist, nehmen somit die Inertie und die Diffusionsdichtigkeit des erfindungsgemäßen Behälters nicht nennenswert ab, wobei der zusätzliche Fertigungsaufwand in engen Grenzen hält.

Die zur Formgebung der thermoplastischen oder duroplastischen Kunststoffe notwendigen Temperaturen liegen deutlich unter denen, die zum Umformen von Glas notwendig sind. Hieraus ergeben sich weitere Vorteile gegenüber reinen Glasbehältern. Der Energiebedarf für den Herstellungsprozess ist aufgrund der niedrigeren Temperaturen gegenüber herkömmlichen Glasspritzen deutlich geringer. Aber auch gegenüber Kunststoffspritzen wird der Energiebedarf verringert, da die Anschlusskörper im Vergleich zu ganzen Kunststoffspritzen kleiner sind und somit weniger Kunststoffvolumen erwärmt und umgeformt werden muss, woraus sich auch eine Materialersparnis ergibt.

Da die Anschlusskörper aus Kunststoff gefertigt werden, wird auch bei komplexen Geometrien kein Wolframstift benötigt, so dass der erfindungsgemäße Behälter keine Wolfram-Rückstände aufweist, die sich nachteilig auf die gelagerte Substanz auswirken könnten. Dies trifft in besonderem Maße auf den Durchtrittskanal zu, der sich mit einem Anschlusskörper aus Kunststoff deutlich einfacher herstellen lässt als aus Glas. Wolframstifte werden nicht benötigt. Generell ist es bevorzugt, wenn die Anschlusskörper aus einem Kunststofftyp bestehen, jedoch ist es auch möglich, dass die Anschlusskörper auch mehrere Kunststofftypen oder auch andere Materialien wie Metall umfassen, beispielsweise zur Verstärkung der Anschlusskörper.

Unter dem distalen Ende soll das Ende des Behälters verstanden werden, welches beim Applizieren der Substanz von der Hand der handhabenden Person weg zeigt. Beim Applizieren durchtritt die Substanz die erste Öffnung und den Durchtrittskanal und kann so den Hohlraum verlassen.

Gemäß der Erfindung ist die Verbindung unlösbar. Unter einer unlösbaren Verbindung soll verstanden werden soll, dass die Verbindung beim bestimmungsgemäßen Gebrauch nicht ohne eine Zerstörung der Verbindung selbst, des Glaskörpers und/oder des Anschlusskörpers gelöst werden kann. In einer nicht zur Erfindung gehörigen Ausführung kann aber auch eine lösbare Verbindung realisiert werden, wozu sich insbesondere der Reibschluss anbietet. Allerdings wird die unlösbare Verbindung vorzugsweise bei Anschlusskörpern eingesetzt, an die Kanülen, Schläuche oder ähnliche Verbindungen angeschlossen werden. Durch die unlösbare Ausgestaltung der Verbindung wird verhindert, dass die Verbindung versehentlich gelöst wird, wodurch die gelagerte Substanz aus dem Behälter austreten und verloren gehen könnte.

Darüber hinaus kann die Verbindung unter Verwendung eines geeigneten Klebstoffs bereitgestellt werden, um den Anschlusskörper aus Kunststoff mit dem Glaskörper zu verbinden. Der Klebstoff kann bei den oben genannten Fügeverfahren auch additiv zum Einsatz kommen, um die Verbindung zu stärken.

In einer bevorzugten Weiterentwicklung des Behälters weist der Glaskörper ein proximales Ende mit einer zweiten Öffnung auf, wobei am distalen Ende ein erster Anschlusskörper und am proximalen Ende ein zweiter Anschlusskörper mit dem Glaskörper verbunden ist. Vorzugsweise weist der Glaskörper im Wesentlichen eine hohlzylindrische Form auf und bildet ein distales Ende mit einer ersten Öffnung und ein proximales Ende mit einer zweiten Öffnung, wobei die Öffnungen von Stirnflächen und von unmittelbar angrenzenden Flächen des Glaskörpers umschlossen werden und der oder die Anschlusskörper über die Stirnflächen und/oder über die unmittelbar angrenzenden Flächen am Glaskörper befestigt sind. In dieser Weiterentwicklung ist der Glaskörper als ein im Wesentlichen rohrförmiger Glaskörper ausgestaltet. Bei den unmittelbar angrenzenden Flächen handelt es sich in diesem Fall vorzugsweise um den Teil der inneren Mantelfläche des Glaskörpers, der sich der Stirnfläche anschließt. Weist der Glaskörper eine hohlzylindrische Form auf, kann er auf einfache Weise kostengünstig gefertigt werden, beispielsweise aus einem Rohrglas. Das Rohrglas wird im Wesentlichen nur noch auf die entsprechende Länge zugeschnitten, um das gewünschte Volumen bereitzustellen, ohne dass Änderungen an den Anschlusskörpern vorgenommen werden müssten. Folglich muss nur die Länge geändert werden, um das Volumen des Hohlraums zu ändern. Es werden daher für unterschiedliche Volumina der Behälter keine weiteren Werkzeuge zum Herstellen der Anschlusskörper benötigt, weshalb die erfindungsgemäßen Behälter flexibel und kostengünstig mit den gewünschten Volumina versehen werden können. Weitere Bearbeitungsschritte am Glaskörper selbst sind nicht notwendig. Allein die Anschlusskörper werden mit der Geometrie versehen, welche für den jeweiligen Gebrauch benötigt wird. Auf diese Weise können die Behälter besonders kostengünstig und in unterschiedlichen Konfigurationen hergestellt werden, da ein Baukastensystem aus verschiedenen Anschlusskörpern realisiert werden kann, wobei die Anschlusskörper über eine einheitliche Schnittstelle mit dem Rohrglas mit wählbarer Länge verbindbar sind.

Dabei kann der Glaskörper aus einer Glasschicht oder mehreren Glasschichten bestehen. Mehrschichtige Glaskörper haben den Vorteil, dass Beschädigungen am Glaskörper nicht unmittelbar zu einer Undichtigkeit führen. Beschädigungen können beispielsweise durch mechanische, chemische oder thermische Einflüsse hervorgerufen werden. Mechanische Einflüsse können beispielsweise vom Benutzer auf den Glaskörper ausgeübte Kräfte oder Momente sein, thermische Einflüsse werden von Temperaturschwankungen insbesondere im Bereich von -20°C bis +40°C hervorgerufen. Beide Einflüsse führen zu einer Rissbildung. Chemische Einflüsse können von der gelagerten Substanz ausgehen und beispielsweise zu einer Delamination führen. Durch den mehrschichtigen Aufbau wird die Rissausbreitung verhindert. Ist eine Schicht nicht mehr dicht, dichtet die andere Schicht den Behälter ab.

Wenn der Anschlusskörper nicht nur über die Stirnfläche, sondern auch über die unmittelbar angrenzenden Flächen mit dem Glaskörper verbunden wird, kann hierdurch die Kontaktfläche vergrößert werden, was es ermöglicht, eine stabilere Verbindung zu schaffen. Dies ist insbesondere bei zähflüssigen Substanzen von Vorteil, bei denen beim Applizieren ein hoher Druck aufgebracht werden muss, dem die Verbindung standhalten muss.

Dabei ist bevorzugt der erste Anschlusskörper als ein Luer-Lock-Anschluss ausgebildet. Luer-Lock-Anschlüsse finden in labortechnischen, medizinischen und pharmazeutischen Anwendungen weite Verbreitung, beispielsweise, um Schläuche oder Kanülen am distalen Ende anzuschließen. Ein Luer-Lock-Anschluss ist ein genormtes Bauteil, welches im Wesentlichen ein Innengewinde mit einer genormten, relativ großen Steigung, und einen koaxial verlaufenden Kegel umfasst. Da der Luer-Lock-Anschluss normgerecht gefertigt werden muss, werden an seine Herstellung hohe Anforderungen bezüglich der Präzision gestellt, weshalb seine Herstellung aus Glas sehr aufwendig ist. Die erforderliche Präzision lässt sich mit Glas schwer bereitstellen, so dass es zu einer relativ hohen Ausschussrate kommt. Neben der aufwendigen Herstellung eignet sich Glas aufgrund seines spröden Werkstoffverhaltens nicht besonders zur Verwendung als Luer-Lock-Anschluss. Erfindungsgemäß kann ein Luer-Lock-Anschluss aus Kunststoff gefertigt und mit dem hohlzylindrischen Glasbehälter verbunden werden, so dass einerseits der Fertigungsprozess vereinfacht werden und andererseits der Luer-Lock-Anschluss aufgrund der höheren Elastizität von Kunststoff gegenüber Glas deutlich höhere Kräfte aufnehmen kann.

In einer besonders bevorzugten Fortbildung des erfindungsgemäßen Behälters ist der zweite Anschlusskörper als ein Fingerflansch ausgebildet. In dieser Fortbildung eignet sich der erfindungsgemäße Behälter besonders für mit einer Substanz vorfüllbare Spritzen, wobei über die zweite Öffnung des proximalen Endes, an welchem der Fingerflansch angeordnet ist, ein Kolben in den hohlzylindrischen Glasbehälter eingebracht werden kann. Der Kolben ist so gefertigt, dass er den Hohlraum gegenüber dem betreffenden proximalen Ende abdichtet, so dass keine Substanz über dieses Ende entweichen kann. Am distalen Ende kann beispielsweise ein Luer-Lock-Anschluss vorgesehen sein, in den geeignete Verschlüsse eingeschraubt werden können, so dass der Behälter auch am distalen Ende abgedichtet ist, um den Austritt der Substanz aus dem Hohlraum zu verhindern. Nach Öffnen des Luer-Lock-Anschlusses kann eine Kanüle angeschlossen werden, so dass die Substanz bequem appliziert werden kann, wozu der Benutzer den Kolben mit dem Daumen in den Hohlraum schieben kann, während sich seine Finger am Fingerflansch abstützen. Der Fingerflansch kann auch eine "Backstop-Funktion" aufweisen, so dass der Kolben nicht versehentlich aus dem Hohlraum entfernt werden kann. Erfindungsgemäß lassen sich derartige vorfüllbare Spritzen einfach und kostengünstig fertigen.

In einer weiteren Ausgestaltung des erfindungsgemäßen Behälters ist zwischen dem Glaskörper und dem Anschlusskörper eine Dichtung vorgesehen, die beabstandet von der Verbindung angeordnet ist. Die Dichtung kann beispielsweise durch einen O-Ring realisiert werden, der eine gewisse Reibkraft zwischen dem Anschlusskörper und dem Glaskörper erzeugt. Neben der dichtenden Wirkung wird die Verbindung zwischen dem Glaskörper und dem Anschlusskörper entlastet. Wird beispielsweise die Substanz mit einem Kolben durch den Anschlusskörper aus dem Hohlraum gedrückt, wird ein Teil der hierdurch auf den Anschlusskörper wirkenden Kräfte über die Dichtung an den Glaskörper übertragen, so dass die Verbindung diesen Teil der Kräfte nicht aufnehmen muss. Folglich kann hierdurch die Wahrscheinlichkeit des Versagens der Verbindung im Gebrauch des erfindungsgemäßen Behälters verringert werden. Zudem kann durch das Vorsehen der Dichtung vermieden werden, dass die Substanz in Kontakt mit der Verbindung gelangt. Hierdurch ist es möglich, auch Klebstoffe zu verwenden, die sonst in die Substanz diffundieren können. Eine derartige Diffusion ist insbesondere bei pharmazeutischen Substanzen nicht akzeptabel. Allerdings können Klebstoffe die Verbindung stabilisieren, so dass in dieser Ausgestaltung die stabilisierende Wirkung von Klebstoffen genutzt werden kann, ohne die nachteilige Diffusion in Kauf nehmen zu müssen.

Vorzugsweise ist der thermoplastische Kunststoff ein Cyclo-Olefin-Copolymer (COC), ein Cyclo-Olefin-Polymer (COP), Acrylnitril-Butadien-Styrol (ABS), Polyamid (PA), Polylactat (PLA), Polymethylmethacrylat (PMMA), Polycarbonat (PC), Polystyrol (PS) oder Polyethylenterephthalat (PET). Der duroplastische Kunststoff ist vorzugsweise ein Celluloseacetat (CA) oder ein transparentes duroplastisches Harz.

Insbesondere die Cyclo-Olefin-Polymere und die Cyclo-Olefin-Copolymere weisen eine hohe Diffusionsdichtigkeit auf, so dass sie sich insbesondere zur Herstellung als Luer-Lock-Anschluss eignen. Da vorgefüllte Spritzen bereits einen Kolben aufweisen, der in den zylindrischen Glaskörper eingebracht ist, ist es nicht notwendig, den Glaskörper an diesem Ende zusätzlich abzudichten. Folglich braucht der Kunststoff, der für den Fingerflansch verwendet wird, keine besonders hohe Diffusionsdichtigkeit aufzuweisen, so dass die übrigen thermoplastischen und duroplastischen Kunststoffe zur Herstellung des Fingerflansches geeignet sind. Aufgrund von gesetzlichen Bestimmungen muss der Luer-Lock-Anschluss transparent sein, damit die Substanz von Außen gut sichtbar ist. Alle genannten Kunststoffe können transparent bereitgestellt werden. Der Fingerflansch hingegen kann aber auch mit einer Farbe versehen werden, um dem Arzt oder der Krankenschwester die Zuordnung verschiedener Spritzen zu erleichtern.

Die genannten Kunststoffe sind gut erforscht, so dass der Anschlusskörper je nach Anwendungsgebiet aus dem hierfür jeweils geeigneten Kunststoff gefertigt werden kann, wodurch sich eine große Produktvielfalt mit relativ geringem Aufwand erzielen lässt.

Ein weiterer Aspekt der vorliegenden Erfindung betrifft die Verwendung eines Behälters nach einem der vorherigen Ausführungsbeispiele zum Lagern und/oder Applizieren einer pharmazeutischen Substanz. Unter einer pharmazeutischen Substanz soll ein Medikament verstanden werden, welches gezielt zur Behandlung des menschlichen oder tierischen Körpers verwendet wird. Die Vorteile, die sich aus der Verwendung eines Behälters nach einem der zuvor genannten Ausführungsbeispiele ergeben, sind insbesondere darin zu sehen, dass der Glaskörper des erfindungsgemäßen Behälters keinem Heißumformprozess unterzogen werden muss, um ihn an bestimmten Stellen eine gewünschte geometrische Form zu geben. Insbesondere muss auch kein Wolframstift verwendet werden, um dünne Kanäle zu formen. Folglich weist der erfindungsgemäße Glaskörper auch keine Wolfram-Rückstände auf, welche in die gelagerte Substanz migrieren könnten. Ferner können die Anschlusskörper ohne großen Aufwand mit komplexen Geometrien versehen werden. Insofern eignet sich der erfindungsgemäße Behälter besonders als pharmazeutisches Primärpackmittel wie vorfüllbare Spritzen. Darüber hinaus sind vorfüllbare Spritzen besonders schnell einsetzbar, da die Substanz nicht von einem Behälter in einen anderen Behälter umgefüllt werden muss. Durch das Wegfallen des Umfüllens reduziert sich auch das Infektions- und Kontaminationsrisiko, was ein wesentlicher Vorteil ist.

Die Erfindung wird im Folgenden anhand eines bevorzugten Ausführungsbeispiels im Detail erläutert.

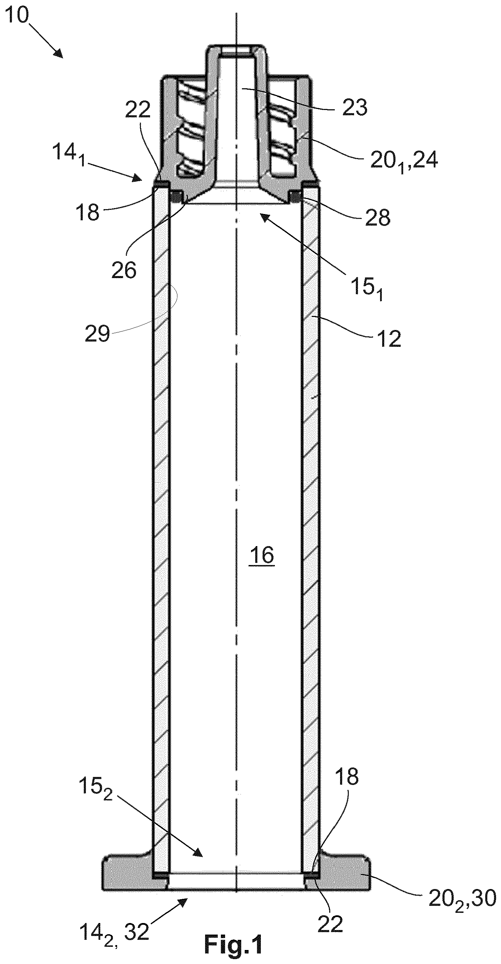

- Figur 1: zeigt eine bevorzugte Ausführungsform des erfindungsgemäßen Behälters anhand einer Schnittdarstellung.

Der in Figur 1 dargestellte erfindungsgemäße Behälter 10 umfasst einen Glaskörper 12, der im dargestellten Ausführungsbeispiel eine hohlzylindrische Form mit einem distalen Ende 14₁ mit einer ersten Öffnung 15₁ und einem proximalen Ende 142 mit einer zweiten Öffnung 15₂ aufweist. Der Glaskörper 12 umschließt einen Hohlraum 16, in welchem eine nicht dargestellte Substanz, beispielsweise eine pharmazeutische Substanz, gelagert werden kann. An jedem der Enden 14₁, 14₂ weist der Glaskörper 12 jeweils eine Stirnfläche 18 auf. Am distalen Ende 14₁ ist ein erster Anschlusskörper 20₁ und am proximalen Ende 14₂ ein zweiter Anschlusskörper 20₂ mit je einer mittels eines Fügeverfahrens bereitgestellten Verbindung 22 mit dem Glaskörper 12 verbunden, wobei die Verbindung 22 auf den Stirnflächen 18 des Glaskörpers 12 angeordnet ist. Die Anschlusskörper 20 bestehen aus einem thermoplastischen oder duroplastischen Kunststoff oder umfassen diese Art von Kunststoffen, welche die Verbindung 22 mit dem Glaskörper 12 ermöglicht. Der am distalen Ende 14₁ angeordnete erste Anschlusskörper 20₁ weist einen Durchtrittskanal 23 auf, der mit der ersten Öffnung 15₁ in Kommunikation steht, so dass die Substanz durch die erste Öffnung 15₁ und den Durchtrittskanal 23 aus dem Hohlraum 16 hinaus in die Umgebung des Behälters 10 befördert werden kann. Dabei ist der Durchtrittskanal 23 wahlweise verschließbar, um das unkontrollierte Austreten der Substanz aus dem Hohlraum 16 zu verhindern.

Im dargestellten Beispiel ist der erste Anschlusskörper 20₁ als ein Luer-Lock-Anschluss 24 ausgebildet, wobei der Luer-Lock-Anschluss 24 einen Vorsprung 26 aufweist, der in den Hohlraum 16 des Behälters 10 hineinragt. Zwischen dem Vorsprung 26 und dem Glaskörper 12 ist eine Dichtung 28 angebracht, um den Luer-Lock-Anschluss 24 gegenüber dem Glaskörper 12 und somit den Hohlraum 16 am distalen Ende 14₁ abzudichten und die Verbindung 22 zwischen dem Luer-Lock-Anschluss 24 und dem Glaskörper 12 zu unterstützen. Die Dichtung 28 wirkt dabei mit einer unmittelbar an die Stirnfläche 18 angrenzende Fläche 29, in diesem Fall mit der inneren Mantelfläche des Glaskörpers 12 zusammen.

Am proximalen Ende 14₂ ist der zweite Anschlusskörper 20₂ als ein Fingerflansch 30 ausgebildet, der selbst eine Öffnung 32 aufweist, die im Wesentlichen dieselbe Querschnittsform und -fläche aufweist wie die zweite Öffnung 15₂ des proximalen Ende 14₂. Durch die Öffnung 32 und die zweite Öffnung 15₂ des proximalen Endes 14₂ kann ein hier nicht dargestellter Kolben in den Hohlraum 16 eingebracht werden. Eine Dichtung wie am distalen Ende 14₁ ist in diesem Fall nicht notwendig, da der Kolben selbst dichtend mit dem Glaskörper 12 abschließt, so dass der Hohlraum 16 auch gegenüber dem proximalen Ende 14₂ abgedichtet ist. Der Fingerflansch 30 kann alternativ etwas über den Glaskörper 12 radial nach innen überstehen, so dass eine Backstop-Funktion realisiert wird und der Kolben nicht unbeabsichtigt aus dem Hohlraum 16 entfernt werden kann.

In der dargestellten Ausführungsform eignet sich der Behälter 10 insbesondere als vorfüllbare Spritze, so dass eine Substanz, insbesondere eine pharmazeutische Substanz, vom Hersteller in der gewünschten Menge in den Hohlraum 16 eingebracht und dort gelagert wird, transportiert und bei Bedarf appliziert werden kann. Die Substanz kann beispielsweise dadurch appliziert werden, dass eine Kanüle am Luer-Lock-Anschluss 24 angeschlossen wird, mit der die Haut eines zu behandelnden menschlichen oder tierischen Körpers durchstochen werden kann. Durch Drücken des Kolbens in den Hohlraum 16 wird die Substanz über die erste Öffnung 15₁ des distalen Endes 14₁, den Durchtrittskanal 23 und die Kanüle in den zu behandelnden Körper transportiert. Dabei beziehen sich die Bezeichnungen distal und proximal auf die Stellung der Spritze beim Applizieren der Substanz in den Körper des Patienten aus Sicht der handhabenden Person. Die Substanz wird beim Applizieren vom proximalen Ende 14 zum distalen Ende 14₁ gefördert.

### Bezugszeichenliste

- 10: Behälter
- 12: Glaskörper
- 14₁: distales Ende
- 14₂: proximales Ende
- 15₁: erste Öffnung
- 15₂: zweite Öffnung
- 16: Hohlraum
- 18: Stirnfläche

- 20, 20₁, 20₂: Anschlusskörper
- 22: Verbindung
- 23: Durchtrittskanal
- 24: Luer-Lock-Anschluss
- 26: Vorsprung
- 28: Dichtung
- 29: angrenzende Fläche

- 30: Fingerflansch
- 32: Öffnung

## Patentansprüche

1. Behälter zum Lagern und/oder Applizieren einer Substanz, umfassend
- einen Glaskörper (12), der eine im Wesentlichen hohlzylindrische Form aufweist und einen Hohlraum (16) umschließt und ein distales Ende (14₁) mit einer ersten Öffnung (15₁) und ein proximales Ende (14₂) mit einer zweiten Öffnung (15₂) aufweist, und
- einen oder mehrere am Glaskörper (12) angebrachte Anschlusskörper (20), die aus einem Kunststoff bestehen oder diesen umfassen, wobei einer der Anschlusskörper (20) am distalen Ende (14₁) angeordnet ist, wobei
- der Kunststoff ein thermoplastischer oder duroplastischer Kunststoff ist und der Glaskörper (12) und der oder die Anschlusskörper (20) unter Verwendung einer mittels eines Fügeverfahrens bereitgestellten Verbindung (22) miteinander verbunden sind,
- der am distalen Ende (14₁) angeordnete Anschlusskörper (20) einen mit der ersten Öffnung (15₁) kommunizierenden Durchtrittskanal (23) umfasst, und
- die Öffnungen (15₁, 15₂) von Stirnflächen (18) und von unmittelbar angrenzenden Flächen (29) des Glaskörpers (12) umschlossen werden und der oder die Anschlusskörper (20) über die Stirnflächen (18) und über die unmittelbar angrenzenden Flächen (29) am Glaskörper (12) befestigt sind, **dadurch gekennzeichnet, dass** die Verbindung (22) unlösbar ist und dass die unmittelbar angrenzenden Flächen (29) der Teil der inneren Mantelfläche des Glaskörpers (12) sind, der sich den Stirnflächen (18) anschließt.

2. Behälter nach Anspruch 1,
**dadurch gekennzeichnet, dass** am distalen Ende (14₁) ein erster Anschlusskörper (20₁) und am proximalen Ende (14₂) ein zweiter Anschlusskörper (20₂) mit dem Glaskörper (12) verbunden ist.

3. Behälter nach Anspruch 2,
**dadurch gekennzeichnet, dass** der erste Anschlusskörper (20₁) als ein Luer-Lock-Anschluss (24) ausgebildet ist.

4. Behälter nach Anspruch 2 und 3,
**dadurch gekennzeichnet, dass** der zweite Anschlusskörper (20₂) als ein Fingerflansch (30) ausgebildet ist.

5. Behälter nach einem der vorherigen Ansprüche,
**dadurch gekennzeichnet, dass** zwischen dem Glaskörper (12) und dem Anschlusskörper (20) eine Dichtung (28) vorgesehen ist, die beabstandet von der Verbindung (22) angeordnet ist.

6. Behälter nach einem der vorherigen Ansprüche,
**dadurch gekennzeichnet, dass** der thermoplastische Kunststoff ein Cyclo-Olefin-Copolymer (COC), Cyclo-Olefin-Polymer (COP), Acrylnitril-Butadien-Styrol (ABS), Polyamid (PA), Polylactat (PLA), Polymethylmethacrylat (PMMA), Polycarbonat (PC) oder Polyethylenterephthalat (PET) ist.

7. Behälter nach einem der vorherigen Ansprüche,
**dadurch gekennzeichnet, dass** der duroplastische Kunststoff ein Celluloseacetat (CA) oder ein transparentes duroplastisches Harz ist.

8. Verwendung eines Behälters nach einem der Ansprüche 1 bis 7 zum Lagern und/oder Applizieren einer pharmazeutischen Substanz.

## Claims

1. Container for storing and/or applying a substance, comprising:
- a glass body (12), which has a substantially hollow cylindrical shape and encloses a cavity (16), and which has a distal end (14₁) with a first opening (15₁) and a proximal end (14₂) with a second opening (15₂), and
- one or more attachment bodies (20) which are mounted on the glass body (12) and which consist of a plastic or comprise same, wherein one of the attachment bodies (20) is arranged at the distal end (14₁), wherein
- the plastic is a thermoplastic or thermosetting plastic, and the glass body (12) and the one or more attachment bodies (20) are connected to each other using a connection (22) made available by a joining method,
- the attachment body (20) arranged at the distal end (14₁) comprises a through-channel (23) communicating with the first opening (15₁), and
- the openings (15₁, 15₂) are enclosed by end faces (18) and by directly adjacent surfaces (29) of the glass body (12), and the one or more attachment bodies (20) are secured on the glass body (12) via the end faces (18) and via the directly adjacent surfaces (29),
**characterized in that** the connection (22) is non-releasable, and **in that** the directly adjacent surfaces (29) are the part of the inner circumferential surface of the glass body (12) that adjoins the end faces (18).

2. Container according to Claim 1, **characterized in that** a first attachment body (20₁) is connected to the glass body (12) at the distal end (14₁) and a second attachment body (20₂) is attached to the glass body (12) at the proximal end (14₂).

3. Container according to Claim 2, **characterized in that** the first attachment body (20₁) is configured as a Luer lock connector (24).

4. Container according to Claims 2 and 3, **characterized in that** the second attachment body (20₂) is configured as a finger flange (30).

5. Container according to one of the preceding claims, **characterized in that** a seal (28) is provided between the glass body (12) and the attachment body (20), said seal (28) being spaced apart from the connection (22).

6. Container according to one of the preceding claims, **characterized in that** the thermoplastic material is a cyclo-olefin copolymer (COC), a cyclo-olefin polymer (COP), acrylonitrile-butadiene-styrene (ABS), polyamide (PA), polylactate (PLA), polymethylmethacrylate (PMMA), polycarbonate (PC) or polyethylene terephthalate (PET).

7. Container according to one of the preceding claims, **characterized in that** the thermosetting plastic is a cellulose acetate (CA) or a transparent thermosetting resin.

8. Use of a container according to one of Claims 1 to 7 for storing and/or applying a pharmaceutical substance.

## Revendications

1. Récipient destiné à stocker et/ou à appliquer un produit comprenant :
- un corps en verre (12) ayant essentiellement la forme d'un cylindre creux, renfermant un volume creux (16), et comprenant une extrémité distale (14₁) ayant une première ouverture (15₁) et une extrémité proximale (14₂) ayant une seconde ouverture (15₂), et
- au moins un corps de fermeture (20) monté sur le corps en verre (12) et qui est réalisé en un matériau synthétique ou renferme un matériau synthétique, l'un des corps de fermeture (20) étant monté à l'extrémité distale (14₁), le matériau synthétique étant un matériau synthétique thermoplastique ou un matériau synthétique thermodurcissable, et, le corps en verre (12) et le(les) corps de fermeture (20) étant liés en utilisant une liaison (22) obtenue au moyen d'un procédé d'assemblage,
- le corps de fermeture (20) situé à l'extrémité distale (14₁) comprenant un canal de passage (23) communiquant avec la première ouverture (15₁), et les ouvertures (15₁, 15₂) étant entourées par les surfaces frontales (18) et les surfaces (29) directement voisines du corps en verre (12), et, le(les) corps de fermeture (20) étant fixés sur le corps en verr (12) par l'intermédiaire des surfaces frontales (18) et des surfaces (29) directement voisines,
**caractérisé en ce que**
la liaison (22) est inamovible et les surfaces (29) directement voisines sont la partie de la surface enveloppe interne du corps en verre (12) qui se raccorde aux surfaces frontales (18).

2. Récipient conforme à la revendication 1,
**caractérisé en ce qu'**
à l'extrémité distale (14₁) un premier corps de fermeture (20₁) est relié au corps en verre (12) et à l'extrémité proximale (14₂) un second corps de fermeture (20₂) est relié au corps en verre (12).

3. Récipient conforme à la revendication 2,
**caractérisé en ce que**
le premier corps de fermeture (20₁) est réalisé sous la forme d'un raccord Luer-Lock (24).

4. Récipient conforme à la revendication 2 ou 3,
**caractérisé en ce que**
le second corps de fermeture (20₂) est réalisé sous la forme d'une bride de préhension (30).

5. Récipient conforme à l'une des revendications précédentes,
**caractérisé en ce qu'**
il est prévu, entre le corps en verre (12) et le corps de fermeture (20) un joint d'étanchéité (28) qui est monté à distance de la liaison (22).

6. Récipient conforme à l'une des revendications précédentes,
**caractérisé en ce que**
le matériau synthétique thermoplastique est un copolymère cyclo-oléfinique (COC), un polymère cyclo-oléfinique (COP), un copolymère acrylonitrile-butadiène-styrène (ABS), un polyamide (PA), un polylactate (PLA), un poly-méthyl méthacrylate (PMMA), un polycarbonate (PC) ou un polyéthylène téréphtalate (PET).

7. Récipient conforme à l'une des revendications précédentes,
**caractérisé en ce que**
le matériau synthétique thermodurcissable est de l'acétate de cellulose (CA) ou une résine thermodurcissable transparente.

8. Utilisation d'un récipient conforme à l'une des revendications 1 à 7, pour stocker et/ou appliquer un produit pharmaceutique.
